# EUROPÄISCHE PATENTANMELDUNG

(11) **EP 2 574 276 A1**
(43) Veröffentlichungstag der Anmeldung: **03.04.2013**
(21) Anmeldenummer: 12186050.6
(22) Anmeldetag: 26.09.2012
(51) Int. Cl.: A61B 5/0215, A61B 5/028

(54) **Vorrichtung zur hämodynamischen Überwachung**

(30) Priorität: 30.09.2011 DE 102011114666
(71) Anmelder: Pulsion Medical Systems SE, 85622 Feldkirchen (DE)
(72) Erfinder: Huber, Wolfgang, 83703 Gmund (DE); Joeken, Stephan, 79650 Schopfheim (DE); Peterreins, Martin, 82054 Sauerlach (DE)
(74) Vertreter: Ettmayr, Andreas

(57) **Zusammenfassung**

Zwischen einem zu einem ersten Zeitpunkt bestimmten n-Tupel mit n Komponenten, und mindestens einem zu mindestens einem entsprechenden späteren Zeitpunkt bestimmten n-Tupel mit n Komponenten wird eine Relation gebildet, wobei n eine natürliche Zahl größer oder gleich 1 ist, und die Komponenten mindestens einen bestimmten Parameter und/oder einen eingelesenen Datenwert umfassen. Erfüllt diese Relation ein vorgegebenes Kalibrierkriterium, wird ein Kalibrierungssignal ausgelöst, das angezeigt wird und/oder automatisch eine Rekalibrierung der Vorrichtung zur hämodynamischen Überwachung auslöst. Beispielsweise wird aus der arteriellen Druckkurve das Pulskontur-Herzzeitvolumen PCCO als konstituierende Komponente eines 1-Tupels bestimmt. Solange dieses vom Referenz-Herzzeitvolumen CO_{Ref} um weniger als einen vorgegebenen Schwellenwert, z.B. 10% oder 15% des Referenz-Herzzeitvolumens, abweicht, erfolgt die weitere Paramaterbestimmung ohne neuerliche Kalibrierung. Übersteigt die Abweichung PCCO - CO_{Ref}| dagegen den Schwellenwert, wird das Kalibrierungssignal ausgelöst.

## Beschreibung

### Technisches Gebiet

Die vorliegende Erfindung betrifft im Allgemeinen die hämodynamische Überwachung von Patienten. Im Speziellen betrifft die vorliegende Erfindung eine Vorrichtung zur hämodynamischen Überwachung mit Einlesemitteln zum wiederholten Einlesen von Daten, die mindestens eine physische Größe darstellen, Berechnungsmitteln zum Berechnen mindestens eines Parameters aus den eingelesenen Daten und Kalibriermitteln zum Kalibrieren der Vorrichtung

### Stand der Technik

Es sind verschiedene, zur hämodynamischen Überwachung eines Patienten genutzte Systeme bekannt, wobei im klinischen Kontext zwischen einer hämodynamischen Basis-Überwachung (umfassend Elektrokardiographie (EKG), Pulsoximetrie, kontinuierliche oder intermittierende Blutdruckmessung sowie zentralvenöse Druckmessung (CVP)) und einer erweiterten hämodynamischen Überwachung unterschieden wird. Letztere umfasst unter anderem die Erfassung und Bestimmung globaler Herz-Kreislaufparameter wie des Herzzeitvolumens (Cardiac Output CO), der zentralvenösen Sauerstoffsättigung (Scv02), sowie der kardialen Vor und Nachlast. Methodologisch stehen unter anderem die Verfahren der Oximetrie, der Pulskontur-Herzzeüvolumen-Bestimmung (PCCO), der transösophagealen Echokardiographie, der Pulmonalarterien-Katherisierung zur Verfügung, sowie die mittels verschiedener Indikatorverdünnungsmethoden erfolgende Bestimmung des Herzzeitvolumens (z.B. transpulmonale Thermodilution, Lithium-Dilution, sowie nicht-invasive (z.B. CO₂-basierte) Methoden).

Während die Erfindung prinzipiell keinen Beschränkungen in Bezug auf ihre Anwendung in verschiedenen, zur hämodynamischen Überwachung eines Patienten genutzten Systemen unterliegt, betrifft die Erfindung insbesondere die Kalibrierung oder Rekalibrierung von Patientenmonitoren im Rahmen einer hämodynamischen Überwachung durch vorzugsweise kathetervermittelte Druck- und/oder Temperaturmessung, z.B. mittels Pulskonturanalyse und/oder Thermodilutionsmessung. Solche Patientenmonitore sind in verschiedenen Ausführungsformen aus dem Stand der Technik bekannt. Kathetervermittelte Druck- und/oder Temperaturmessungen dienen dabei der Bestimmung hämodynamischer Parameter wie z.B. des mittleren arteriellen Drucks (MAP), des Herzzeitvolumens (CO), des globalen enddiastolischen Volumens (GEDV) und des extravasalen Lungenwassers (EVLW).

Die Analyse der kontinuierlich über eine arterielle Druckmessung aufgezeichneten Pulskontur erlaubt es, für jede Herzaktion das Schlagvolumen (SV) zu ermitteln, indem aus dem Verlauf (der "Form") einer beispielsweise in einer Beinarterie fortlaufend gemessenen Blutdruckkurve durch mathematische Verfahren das Schlagvolumen des Herzens berechnet wird. Der in der peripheren Arterie gemessene Druck entspricht dabei näherungsweise dem Aortendruck. Grundlage der mathematischen Verfahren ist die Extraktion und klinisch nutzbare Darstellung der in der arteriellen Blutdruckkurve enthaltenen Informationen. Aus derjenigen Fläche unter der Druckkurve, welche oberhalb des diastolischen Druckes liegt und dem Zeitraum der Öffnung der Aortenklappe entspricht, wird das Herzzeitvolumen berechnet. Aus der Pulskonturanalyse können damit auch die respiratorisch bedingten Veränderungen des Schlagvolumens (Schlagvolumenvariation SVV) über den Atemzyklus ermittelt werden, wobei die SW durch eine sich verändernde Vorlast entsteht und somit zur Abschätzung der Volumenreagibilität des linken Ventrikels herangezogen werden kann.

Die Bestimmung des Herzzeitvolumens (CO) mittels Pulskonturanalyse auf Basis eines nichtlinearen Windkesselmodells ist in EP 0 947 941 B1 beschrieben.

Die Kalibrierung des Pulskontur-Analyse Systems erfolgt zumeist mittels Indikator- oder Thermodilution. Die verschiedenen kommerziell verfügbaren Thermodilutionssysteme arbeiten meist mit einem Kälte-Indikator, i.e. einem gekühlten Bolus. Bei der transpulmonalen Thermodilutionsmessung wird eine definierte Menge an kalter Flüssigkeit venös in den Probanden injiziert und der Temperaturverlauf des Blutes transpulmonal mittels einer in einer peripheren Arterie (z.B. Femoralarterie oder Radialarterie) lokalisierten Thermosonde aufgezeichnet. Zur Temperaturmessung bei Thermodilutionsverfahren wird in der Regel ein Thermistor, d.h. ein Widerstandstemperatursensor (RTD, Resistance Temperature Detector) verwendet. RTDs sind aufgrund ihrer Stabilität und der großen Genauigkeit beliebt und weisen ein weitestgehend lineares Messsignal auf.

Verfahren und Vorrichtungen zur transpulmonalen Thermodilutionsmessung sind unter anderem in US 5,526,817 und US 6,394,961 offenbart. In der US 5,526,817 ist ein Verfahren zum Bestimmen des Kreislauffüllungszustandes mittels Thermodilution beschrieben, wobei zur Beurteilung des Kreislauffüllungszustandes eines Patienten verschiedene Volumina und Volumenströme bestimmt werden, insbesondere das globale enddiastolische Volumen (GEDV), das intrathorakale Blutvolumen (ITBV), das pulmonale Blutvolumen (PBV), das extravasale Lungenwasser (EVLW), das intrathorakale Thermovolumen (ITTV), das pulmonale Thermovolumen (PTV) sowie der globale Herzfunktionsindex (CFI).

Wie oben erwähnt, werden Thermodilutions- und Pulskonturverfahren oft kombiniert, wodurch vorteilhaft die Thermodilutionsmessung zur Kalibrierung des Pulskonturverfahrens herangezogen werden kann. Nach der initialen Kalibrierung werden dabei für den klinischen Alltag 2-3 Kalibrierungen pro Tag als übliches Intervall für die Rekalibrierung angeben, um eine hinreichend aussagekräftige kontinuierliche CO-Messung mittels Pulskonturanalyse zu gewährleisten, da die Stabilität bekannter Pulskonturalgorithmen nicht unter allen Umständen gewährleistet ist. Durch das lange Intervall wird zwar zum einen der Ressourcen- und Personalaufwand herabgesetzt und gegebenenfalls eine mit der Thermodilutionsmessung verbundene Volumenbelastung vermieden, andererseits kann ein zu großer Zeitabstand zwischen den Rekalibrierungen zu klinischen Bewertungsfehlem führen, da im Intervall auftretende Veränderungen, wie z.B. Variationen der klinischen Situation, Katheter-Dislokation, oder Arrhythmien nicht notwendigerweise zu einer Rekalibratierung des Systems führen.

### Darstellung der Erfindung

Ausgehend von den vorgenannten Vorrichtungen und Verfahren des Standes der Technik liegt der vorliegenden Erfindung daher die Aufgabe zugrunde, eine Vorrichtung und ein Verfahren zur hämodynamischen Überwachung eines Patienten bereitzustellen, welche die genannten Nachteile nicht aufweisen oder deutlich mindern.

Gelöst wird diese Aufgabe durch eine bedarfsangepasste automatische oder manuelle Kalibrierung bzw. Rekalibrierung bei hämodynamischer Überwachung eines Patienten.

In einem ersten Aspekt stellt die vorliegende Erfindung daher eine Vorrichtung zur hämodynamischen Überwachung eines Patienten bereit, welche Einlesemittel zum wiederholten Einlesen von Daten, die mindestens eine physische Größe darstellen, Berechnungsmittel zum Berechnen mindestens eines Parameters mit Hilfe der eingelesenen Daten, und Kalibriermittel zum Kalibrieren der Vorrichtung aufweist und ferner Auslösemittel zum Auslösen eines Kalibrierungssignals besitzt. Dabei sind die Auslösemittel geeignet, das Kalibrierungssignal in Abhängigkeit der zeitlichen Änderung der eingelesenen Daten und/oder mindestens eines der Parameter auszulösen. Die vorliegende Erfindung stellt somit eine Vorrichtung zur hämodynamischen Überwachung eines Patienten bereit, welche ein gegenüber dem Stand der Technik deutlich verbesserte Rekalibrierungsprocedere ermöglicht. Die erfindungsgemäße Rekalibrierungstechnik stellt dabei auf die tatsächlichen Veränderungen des Patienten ab, so dass eine Kalibrierung zuverlässig dann initiiert werden kann, wenn sich eine vom vorherigen Zustand des Patienten signifikante Abweichung der hämodynamischen Gesamtsituation ergibt. Durch die erfindungsgemäße Vorrichtung und das entsprechende Verfahren werden unnötige Messungen und eine z.B. im Rahmen einer wiederholten Thermodilutionsmessung auftretende unnötige Volumenbelastung vermieden und der technische und personelle Aufwand nicht unnötig erhöht. Erfindungsgemäße Vorrichtungen und Verfahren können damit ferner eine bessere Aussagekraft in Bezug auf reale Veränderungen ermöglichen.

In einer bevorzugten Implementierung kann das Auslösemittel dazu ausgebildet sein, einen n-Tupel mit n Komponenten zu bestimmen, welche mindestens einen Parameter und/oder einen Wert der eingelesenen Daten einschließen, wobei n eine natürliche Zahl größer oder gleich 1 ist. Bevorzugt können Tupel mit n>1 Komponenten (n>1 Dimensionen) zur Anwendung kommen. Ferner können die Auslösemittel mittels eines uni- oder multivariaten Analyseverfahrens eine Relation bilden, vorzugsweise eine arithmetische Relation, zwischen einem zu einem ersten Zeitpunkt bestimmten n-Tupel, und mindestens einem zu mindestens einem späteren Zeitpunkt bestimmten n-Tupel mit jeweils n Komponenten. Vorteilhaft können insbesondere bei Tupeln mit n>1 Dimensionen multivariante Analyseverfahren zur Anwendung kommen. Bei entsprechender Datenreduktion oder Gewichtung können aber auch univariate Verfahren verwendet werden. Bevorzugt kann eine Differenz bestimmt werden zwischen dem zu einem ersten Zeitpunkt bestimmten n-Tupel und mindestens einem zu mindestens einem späteren Zeitpunkt bestimmten n-Tupel. Grundsätzlich kann eine Differenz z.B. im Rahmen einer Verlaufsbestimmung, über einen durch mehrere Zeitpunkte definierten Zeitraum bestimmt werden, bevorzugt kann die Differenz zwischen einem ersten und einem zweiten Zeitpunkt bestimmt werden.

In einer weiteren bevorzugten Ausführungsform sind die Auslösemittel zur Durchführung einer mathematischen Klassifizierungsmethode ausgebildet. Vorzugsweiseweise kann die Klassifizierungsmethode in Form einer *Support Vector Machine* implementiert sein, oder es sich um eine Diskriminanzanalyse handeln. Unter einer Diskriminanzanalyse wird eine Methode der multivariaten statistischen Verfahren verstanden, welche der Unterscheidung von zwei oder mehreren Gruppen dient. Die Gruppen werden mit mehreren Merkmalen (auch Variablen) beschrieben. In der erfindungsgemäßen Vorrichtung kann dabei durch die Auslösemittel die Signifikanz der aus der Diskriminanzanalyse ermittelten Diskriminanzgröße Yₘ bestimmt werden. Weicht die aus einer ersten und aus einer nachfolgenden zweiten Bestimmung ermittelte Diskriminanzgröße Ym um eine vorgegebene Abweichungsgröße von der Diskriminanzgröße Y_{Kal}) ab, welche in einem zeitlich naheliegenden Abstand zu einer Kalibrierung ermittelt wurde, kann durch die Auslösemittel ein Kalibrierungssignal ausgelöst werden. Abweichungsgrößen können dabei absolut oder prozentual zu einem vorher festgelegten Wert in Beziehung gesetzt werden. Eine vorteilhafte Abweichungsgröße ist eine Abweichung der zuletzt bestimmten Diskriminanzgröße Ym um 5% bis 15% von der Diskriminanzgröße Y_{Kal}. Alternativ können auch direkt aufeinanderfolgend bestimmte Diskriminanzgrößen Ym und Yₘ₊₁ zueinander mittels Diskriminanzanalyse in Beziehung gesetzt werden.

Vor der initialen Kalibrierung, bevorzugt mittels Thermodilution, kann von den Auslösemitteln ein n-Tupel mit nur n-1 oder n-(>1) Komponenten bestimmt werden, da (noch) keine Parameter und/oder Größen aus einer vorausgehenden Kalibrierung vorliegen. Weicht die von den Auslösemitteln aus einer ersten und aus einer nachfolgenden zweiten Bestimmung ermittelte Diskriminanzgröße Y_{Kal} um eine vorgegebene Abweichungsgröße von einer vorgegebenen Referenzgröße Y_{R} ab, kann ein Kalibrierungssignal ausgelöst werden, wobei es sich bei der Referenzgröße Y_{R} um eine (insb. initial) gemessene Größe oder einen anderweitig vorgegebenen (als Standardwert vorgespeicherten oder eingegebenen) Vergleichswert handeln kann. Das um n-1 oder n-(>1) Komponenten verkleinerte n-Tupel kann so zudem als Indikator für einen Systemwechsel dienen.

In einer weiteren vorteilhaften Ausführungsform der efindungsgemäßen Vorrichtung können die Auslösemittel zur Durchführung einer linearen Diskriminanzanalyse ausgebildet sein. Während grundsätzlich z.B. auch die Varianten der quadratischen, regularisierten, diagonalen oder Nearest-Centroid-Diskriminanzanalyse zur Anwendung kommen können, bietet die lineare Diskriminanzanalyse den Vorteil der einfachen Durchführbarkeit bei nur geringer Modellkomplexität. Ferner können vorteilhaft normalverteilte Datensätze mit weitgehend korrespondierenden Kovarianzmatrizen zur linearen Diskriminanzanalyse herangezogen werden. Dadurch können insbesondere die Entscheidungsgrenzen für benutzte Datensätze (Rekalibrierung ja/nein) präzise definiert werden.

Vorzugsweise kann der n-Tupel mit n Komponenten mindestens einen Parameter einschließen, welcher aus der Gruppe Herzfrequenz (HR), Pulsdauer (T), Schlagvolumen (SV), mittlerer arterieller Druck (MAP), Pulskontur-Herzzeitvolumen (PCCO), Thermodilutions-Herzzeitvolumen (CO_{TD}), Schlagvolumenvariation (SW), systemischer Gefäßwiderstand (SVR), Pulsdruckvariation (PPV), Volumenreagibilitätsindex (FRI), ventrikuläre Kontraktilität (z. B. dPmx), atriale Drücke und/oder ventrikuläre Drücke (z.B. RAP, RVEDP, LVEDP), EKG-Abschnittsdaten und EKG-Intervalle sowie Kenndaten der die Hämodynamik beeinflussende Medikation und die Hämodynamik beeinflussende Atmungsparamater ausgewählt ist. Geeignete EKG-Abschnittsdaten und EKG-Intervalle sind dabei z. B. Höhe und Breite der P- Welle, der T-Welle, des QRS Komplexes sowie die Länge des PQ-Intervalls, der ST-Strecke. Ferner können Extrasystolen und andere Herzrhythmusstörungen berücksichtigt werden. Kenndaten der die Hämodynamik beeinflussende Medikation können beispielsweise die Dosis eines bestimmten Medikaments umfassen, die Hämodynamik beeinflussende Atmungsparamater können den positiven endexpiratorischen Druck PEEP, den mittleren Luftwegedruck oder den Beatmungsmodus umfassen.

Besonders bevorzugt kann der n-Tupel mindestens einen Parameter mit möglichst großer absoluter Aussagekraft einschließen, welcher vorzugsweise mittels einer bestehenden Überwachungsvorrichtung nicht- oder minimal-invasiv, i.e. möglichst einfach, gemessen und/oder bestimmt werden kann. Insbesondere können Parameter geeignet sein, deren Bestimmung keine vorherige Kalibrierung erforderlich macht und/oder deren Abweichung von einem Referenz- und/oder vorherigen Wert einfach erkennbar sind. Darunter fallen erfindungsgemäß alle kardiovaskulären Parameter, welche vorteilhaft unabhängig von höherinvasiven Verfahren bestimmbar sind und deren Bestimmung nur kleinen systematischen Fehlern unterliegt wie z. B. die Herzfrequenz, die Pulsdauer, der geschätzte mittlere Arteriendruck und bestimmte EKG-Parameter. Mittels aufwändigerer nicht-invasiver Verfahren bestimmte Parameter, z.B. aus der Langzeitechokardiagraphie oder der Impedanzkardiographie können ebenfalls als Komponenten eines n-dimensional ausgebildeten Tupels fungieren. Insbesondere können mittels der für die hämodynamische Überwachung bevorzugten Messanordnung die Parameter Pulskontur-Herzzeitvolumen (PCCO), systemischer Gefäßwiderstand (SVR) und Thermodilutions-Herzzeitvolumen (CO_{TD}) durch minimal invasive kathetervermittelte Druck- und/oder Temperaturmessung, z.B. mittels Pulskonturanalyse und/oder Thermodilutionsmessung, mit nur geringem Aufwand ermittelt werden.

In einer weiteren Ausführungsform der erfindungsgemäßen Vorrichtung kann der n-Tupel mit n Komponenten mindestens einen Datenwert einschließen ausgewählt aus der Gruppe arterieller Druck (P), zentralvenöser Druck (CVP), Bluttemperatur (Tb), periphere Sauerstoffsättigung (Sp02), zentralvenöse Sauerstoffsättigung (Scv02). Besonders bevorzugt kann der n-Tupel Datenwerte mit möglichst großer absoluter Aussagekraft einschließen, welche vorzugsweise mittels einer bestehenden Überwachungs-Vorrichtung nicht- oder minimal-invasiv, i.e. möglichst einfach, gemessen und/oder bestimmt werden können. Insbesondere können Datenwerte geeignet sein, deren Messung keine vorherige Kalibrierung erforderlich macht und/oder deren Abweichung von einem Referenz- und /oder vorherigen Wert einfach erkennbar sind.

In einer weiteren Ausführungsform der erfindungsgemäßen Vorrichtung kann der zeitliche Abstand zwischen der ersten und der mindestens einen zeitlich späteren Bestimmung des n-Tupels mit n Komponenten unter einer Stunde betragen. Dabei kann der bevorzugte zeitliche Abstand in Abhängigkeit von den Komponenten des n-Tupels variieren, Insbesondere bei einem n-Tupel, dessen Komponenten aus nicht-invasiv messbaren und/oder einfach zu ermittelnden Parameter- und Datenwerten bestehen (z.B. HF, SpO2), kann der Abstandszeitraum zwischen den einzelnen Tupel-Bestimmungen gering gewählt werden. Dementsprechend kann der Abstandszeitraum zwischen den einzelnen Tupel-Bestimmungen größer gewählt werden, wenn die Komponenten des Tupels invasiv messbare und/oder aufwändiger zu ermittelnde Parameter- und Datenwerten enthalten (z.B. Scv02). Besonders bevorzugt ist ein zeitlicher Abstand von höchstens 15 Minuten bis hinunter zu wenigen oder einzelnen Herzschlägen (Beat-to-Beat-Beurteilung ob eine Rekalibrierung erforderlich ist). Insbesondere bei geringen Zeitabständen kann eine kontinuierliche Bestimmung des Parameterraumes erfolgen, bevorzugt bei der Verwendung von Datenwerten und oder Parametern, welche entweder keine initiale Kalibrierung erfordern oder die mit geringem Aufwand bei bestehender Monitoring-Anordnung gemessen und/oder errechnet werden können.

Um die Implementierung zu vereinfachen oder um negative Nebenwirkungen zu häufiger Ausführung invasiver Schritte im Zusammenhang mit einem bestimmten Kalibrierverfahren zu vermeiden, kann es auch vorteilhaft sein, ein vorbestimmtes minimales Zeitintervall zwischen zwei aufeinanderfolgenden Kalibrierungen vorzusehen, z.B. zehn Sekunden, dreißig Sekunden, eine, fünf, zehn, 15 oder 30 Minuten, und/oder eine maximale Anzahl von Kalibrierungen pro Zeiteinheit, z.B. jegliche zwischen zwei und 20 pro Stunde. Derartige minimale Zeitintervalle und maximale Anzahlen an Kalibrierungen pro Zeiteinheit können stark variieren in Abhängigkeit der Umstände der Anwendung der Erfindung, z.B. der Komplexität der Kalibriermethode oder des Grades der Invasivität von in Kombination mit dem Kalibrierverfahren ausgeübten Schritten.

Besonders bevorzugt sind die Auslösemittel dazu ausgebildet, innerhalb eines vorgegebenen Intervalls aufeinanderfolgende gegenläufige zeitliche Änderungen der eingelesenen Daten und/oder mindestens eines der Parameter auf eine Weise zu berücksichtigen, welche (vorzugsweise mittels einer Filterung oder Mittelung) eine geringeren Gewichtung der zu einem Zeitpunkt eingelesenen Daten und/oder des zu einem Zeitpunkt ermittelten Parameters bewirkt, die sowohl von den unmittelbar vorher als auch von den unmittelbar nach diesem Zeitpunkt eingelesenen entsprechenden Daten in dieselbe Richtung abweichen, bzw. der sowohl von dem unmittelbar vorher als auch von dem unmittelbar nach diesem Zeitpunkt ermittelten Parameter in dieselbe Richtung abweicht (sogenannte "Ausreißer"). Die Gewichtung kann bis zu einer völligen Nichtberücksichtigung eines "Ausreißers" mittels eines Werteauswahlalgorithmus herabgesetzt sein, aber beispielsweise auch einfach durch Mittelung über mehrere aufeinanderfolgende Parameter- bzw. Datenwerte erzielt werden. Anschaulich kann die genannte Berücksichtigung aufeinanderfolgende gegenläufige zeitliche Änderungen der eingelesenen Daten und/oder mindestens eines der Parameters als Glättung des zeitlichen Verlaufs der eingelesenen Daten bzw. des eingelesenen Parameters verstanden werden.

In einer weiteren Ausführungsform der erfindungsgemäßen Vorrichtung kann das durch die Auslösemittel ausgelöste Kalibrierungssignal eine automatische Kalibrierung bzw. Rekalibrierung einleiten. Die im klinischen Alltag gebräuchliche manuelle Kalibrierung bzw. Rekalibrierung kann durch eine entsprechende automatische Kalibrierung bzw. Rekalibrierung ersetzt werden. Eine automatische Kalibrierung bzw. Rekalibrierung kann dabei besonders vorteilhaft durchgeführt werden, wenn der Kalibrierungsprozess keine zusätzliche invasive oder nur eine minimal-invasive zusätzliche Maßnahme erfordert. Denkbar sind hier z.B. Bolusinjektionen mittels einer elektronisch angesteuerten Injektionspumpe. Oder aber es kann eine wie in EP 1 236 435 A1 beschriebene Thermodilutionstechnik zum Einsatz kommen, welche ohne Injektion eines Kältebolus auskommt und stattdessen eine mittels eines Hitzegenerators oder Peltier-Kühlelement oder dgl. erzeugte lokale Temperaturabweichung verwendet.

In einer weiteren Ausführungsform kann die Auslösung des Kalibrierungssignals visuell und/oder akustisch angezeigt werden, z.B. mittels eines üblichen Displays bzw. Tongenerators, wobei die Anzeige des Kalibrierungssignal vorzugsweise über das Anzeigen eines ohnehin fortlaufend dargestellten Wertes hinausgeht. D.h. die Displayanzeige ändert sich nicht nur durch das umspringen eines angzeigten Zahlenwerts, sondern die Auslösung des Kalibrierungssignals wird dem Anwender vorteilhafterweise erkennbar gemacht, ohne dass dieser selbst einen Wertevergleich durchführen muss. Die fortlaufende Darstellung eines ohnehin angezeigten Messwerts oder hiervon abgeleiteten Parameters stellt somit keine Ausgabe eines Kalibriersignals im Sinne dieser Ausführungsform der vorliegenden Erfindung dar.

Bei automatisch eingeleiteter Kalibrierung wird der Benutzer vorzugsweise durch die akustische oder visuelle Anzeige hierüber in Kenntnis gesetzt. Gemäß einer alternativen vorteilhaften Ausführungsform kann aber auch der durch die Anzeige aufmerksam gemachte Verwender die automatische Kalibrierung durch manuelles Eingreifen auslösen. Das Kalibrierungssignal signalisiert dann lediglich die Notwendigkeit einer vorzunehmenden Kalibrierung bzw. Rekalibrierung, lässt also vorteilhaft eine Beurteilung der klinischen Situation durch den Verwender zu. Vorteilhaft umsetzbar ist auch eine Weiterbildung, bei welcher der Verwender auch einen individuell angepassten, veränderbaren Zeitabstand vorgeben kann, nach dessen Ablauf eine automatische Kalibrierung ohne weiteres Signal vom Verwender erfolgen soll.

Gemäß einer weiteren vorteilhaften Weiterbildung der Erfindung können die Auslösemittel zur zwei- oder mehrstufigen Prüfung der Abhängigkeit der zeitlichen Änderung der eingelesenen Daten und/oder mindestens eines der Parameter ausgelegt sein. Dabei umfasst die zwei- oder mehrstufige Prüfung die hierarchische Überprüfung mindestens zweier das Auslösen des Kalibrierungssignals bedingender, von der zeitlichen Änderung der eingelesenen Daten und/oder mindestens eines der Parameter abhängender Auslösekriterien. Beispielsweise können die Auslösemittel zunächst überprüfen, ob sich ein bestimmter Parameter oder Messwert innerhalb eines vorbestimmten Zeitraums um mehr als einen vorgegebenen Faktor geändert hat, und wenn dieses Kriterium erfüllt ist, als weiteres Kriterium überprüfen, wie gleichmäßig die Änderung im vorgegebenen Zeitraum erfolgte. Eine besonders gleichmäßige Änderung kann z.B. als Nullliniendrift interpretiert werden und das Kalibriersignal auslösen. Auch andere hierarchische Prüfungen von Auslösekriterien für das Kalibriersignal sind implementierbar.

In einer weiteren bevorzugten Ausführungsform umfasst die Vorrichtung zusätzlich Bewertungsmittel zum Bewerten der arithmetischen Relation zwischen unmittelbar vor einer Kalibrierung und unmittelbar nach einer Kalibrierung eingelesenen Daten und/oder zwischen mindestens einem unmittelbar vor einer Kalibrierung und unmittelbar nach einer Kalibrierung bestimmten Parameter. Unmittelbar kann dabei die letzte Messung bzw. Parameterbestimmung vor bzw. nach der Kalibrierung bedeuten, oder aber auch jeweils eine andere Messung bzw. Parameterbestimmung innerhalb eines Zeitraums vor und nach der Kalibrierung, welcher klein ist, d.h. vorzugsweise weniger als zwei Minuten, besonders bevorzugt weniger als eine Minute beträgt.

Mittels der Bewertungsmittel kann eine Evaluierung der Rekalibrierung bzw. des rekalibrierten hämodynamischen Parameters vorgenommen werden. Beispielsweise können das unmittelbar vor einer Kalibrierung bestimmte Pulskontur-Herzzeitvolumen und das unmittelbar nach der Rekalibrierung bestimmte Pulskontur-Herzzeitvolumen zueinander in Beziehung gesetzt werden. Dabei können die Bewertungsmittel anhand der Größe der Abweichung zwischen beiden Parametern bewerten, ob die in Abhängigkeit von der zeitlichen Änderung der eingelesenen Daten und/oder des mindestens einen Parameters erfolgte Rekalibrierung notwendig war oder nicht. Falls unmittelbar vor und nach der Kalibrierung gemessene Daten bzw. bestimmte Parameter nicht oder nur geringfügig voneinander abweichen, kann dies als Zeichen dafür gewertet werden, dass die übermäßige zeitliche Änderung der eingelesenen Daten und/oder des mindestens einen Parameters physiologische Ursachen hat und nicht auf Mess- oder Rechenungenauigkeiten beruht.

Zusätzlich kann mittels der Bewertungsmittel eine Bewertung der von den Auslösemitteln berücksichtigten Datenwerte oder Parameter im n-Tupel erfolgen. Ist beispielsweise der jeweils relevante hämodynamische Parameter nach erfolgter Rekalibrierung unverändert, ist möglicherweise die von den Auslösemitteln berücksichtigte zeitliche Änderung des Datenwerts oder des mindestens einen Parameters ungeeignet, eine auf den interessierenden hämodynamischen Parameter bezogene Veränderung der Situation des Patienten korrekt zu indizieren. Damit kann die durch die Bewertungsmittel vorgenommene Bewertung sich auch dazu eignen, den Parameter- und Datenraum eines n-Tupels entsprechend zu evaluieren und gegebenenfalls zu modifizieren. Die letztgenannte Evaluation und/oder Modifikation kann beispielsweise mittels entsprechender Algorithmen auch automatisch vorgenommen werden und zu einem "Lernprozess" der Vorrichtung führen (Maschinenlernen). Ein aus n Komponenten bestehender n-Tupel kann anhand der Bewertungsergebnisse beispielsweise durch unterschiedliche Gewichtung einzelner Komponenten verändert werden, oder es können Komponenten hinzugefügt oder weggelassen werden.

In einer weiteren bevorzugten Ausführungsform umfasst die Vorrichtung zusätzlich Eingabemittel zum Eingeben spezifischer Informationen über den Patienten. Die spezifischen Informationen über den Patienten können dabei kategorisierende Informationen und/oder biometrische Informationen enthalten. Insbesondere bei der initialen Kalibrierung können so biometrische Daten zur Bestimmung von Referenzwerten hinzugezogen werden. Aber auch zur Erstellung von n-Tupeln mit n>1 Komponenten ist die Verwendung biometrischer Informationen vorteilhaft, da sie z.B. die Indizierung von eingelesenen Datenwerten und Parametern erlauben und damit z.B. die statistische und/oder klinische Aussagekaft der so indizierten Datenwerte und Parameter erhöht. Ferner kann ein von eingegebener Information abhängiges Kalibrierkriterium etabliert werden. Je nachdem, ob ein Patient einer Kategorie zugehört, welche eine bestimmte Parameteränderung erwarten lässt (etwa weil der Patient während der hämodynamischen Überwachung einer bestimmten therapeutischen Maßnahme unterliegt) oder nicht, kann beispielsweise die Änderung dieses Parameters oder Messwerts von den Auslösemitteln stärker oder weniger stark in die Beurteilung einbezogen werden, ob ein Kalibriersignal auszulösen ist oder nicht. Mit anderen Worten können so erwartbare Änderungen von Parameter- bzw. Messwerten aus der Beurteilung ausgeklammert werden, ob eine Neukalibrierung erforderlich ist. Hat ein Patient beispielsweise fiebersenkende Mittel erhalten, so kann es sinnvoll sein, die gemessene Bluttemperatur in einem die Bluttemperatur normalerweise als Eingangswert berücksichtigenden Beurteilungsalgorithmus, ob ein Kalibriersignal auszulösen ist, unberücksichtigt zu lassen.

In einem weiteren Aspekt betrifft die vorliegende Erfindung ein Verfahren zur hämodynamischen Überwachung eines Patienten, welches das Einlesen von Daten, die mindestens eine physische Größe darstellen, das Berechnen mindestens eines Parameters mit Hilfe der eingelesenen Daten, das Auslösen eines Kalibrierungssignals in Abhängigkeit von der zeitlichen Änderung der eingelesenen Daten und/oder mindestens eines der Parameter, und die (Re-)Kalibrierung der Vorrichtung umfasst.

Gemäß einer bevorzugten Ausführungsform umfasst das Verfahren zusätzlich eine Bewertung der arithmetischen Relation zwischen den eingelesenen Daten und/oder mindestens eines der Parameter unmittelbar vor und unmittelbar nach einer (Re-) Kalibrierung.

Grundsätzlich kann jede im Rahmen der vorliegenden Anmeldung beschriebene bzw. angedeutete Variante der Erfindung besonders vorteilhaft sein, je nach wirtschaftlichen und technischen Bedingungen im Einzelfall. Soweit nichts gegenteiliges dargelegt ist, bzw. soweit grundsätzlich technisch realisierbar, sind einzelne Merkmale der beschriebenen Ausführungsformen austauschbar oder miteinander kombinierbar.

### Kurze Beschreibung der Figuren

Im Folgenden werden beispielhaft und nicht abschließend einige besonders vorteilhafte Ausführungsformen der Erfindung unter Bezugnahme auf die begleitenden Zeichnungen beschrieben. Die besonderen Ausführungsformen dienen insbesondere der Erläuterung des erfinderischen Gedankens, jedoch beschränkt sich die Erfindung nicht allein auf die hier dargestellten Aspekte.
- Figur 1: zeigt eine schematische Ansicht des Herz-Kreislaufsystems eines Patienten unter hämodynamischer Überwachung mittels einer bevorzugten Ausführungsform der erfindungsgemäßen Vorrichtung.
- Figur 2: zeigt das Flussdiagramm einer erfindungsgemäßen Auslösung eines Kalibrierungssignals in für mehrere mögliche Ausführungsformen verallgemeinerter Form.
- Figur 3: zeigt ein Flussdiagramm für die Auslösung eines Kalibrierungssignals gemäß einer Ausführungsform mit der zeitlichen Änderung eines einzelnen Parameters als Kalibrierkriterium.
- Figur 4: zeigt ein Flussdiagramm für die Auslösung eines Kalibrierungssignals gemäß einer weiteren Ausführungsform mit zwei hierarchisch angewendeten Kalibrierkriterien.
- Figur 5: zeigt ein Flussdiagramm für die Bewertung des Kalibrierergebnisses gemäß einer weiteren bevorzugten Ausführungsform der Erfindung.

### Detaillierte Beschreibung bevorzugter Ausführungsbeispiele

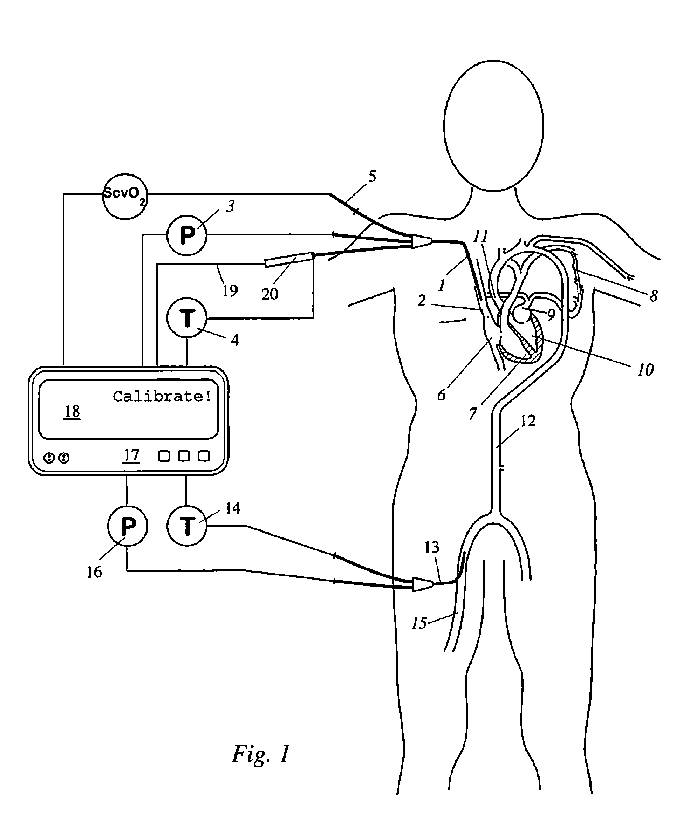

Figur 1 zeigt in schematischer Darstellung die Hauptbestandteile einer bevorzugten Ausführungsform der erfindungsgemäßen Vorrichtung. Ein mehrlumiger Zentralvenenkatheter 1 ist in der vena cava superior 2 eines Patienten lokalisiert. Eine Druckmesslinie 3 mit aus dem Stand der Technik bekanntem Drucksensor dient der kontinuierlichen oder intermittierenden Messung des zentralen Venendrucks. Der Zentralvenenkatheter 1 ist ferner mit einem Injektionskanal 20 mit Injektat-Temperatursensor 4 versehen. Weiterhin weist der Zentralvenenkatheter 1 eine optische Messonde 5 zur Messung der zentralvenösen Sauerstoffsättigung auf.

Für eine Thermodilutionsmessung wird in den Zentralvenenkatheter 1 ein Bolus einer Flüssigkeit injiziert (z.B. 10 ml oder 0,15 ml/kg), welcher als Temperaturindikator dient und eine entweder signifikant wärmere oder kältere Temperatur aufweist als das Blut des Patienten. Durch den Bolus wird im Blutkreislauf des Patienten eine lokale Temperaturabweichung induziert. Diese Temperaturabweichung wandert vom Injektionsort zunächst zum rechten Vorhof 6 und Ventrikel 7, durchläuft daraufhin den Pulmonalkreislauf 8, passiert den linken Vorhof 9 und den linken Ventrikel 10, um danach über die Aorta 11 die systemische Zirkulation 12 zu erreichen. Ein Temperatursensor 14, welcher in einem peripher lokalisierten arteriellen Katheter 13 angeordnet ist, misst die lokale Temperaturabweichung in Abhängigkeit von der Zeit. So kann jede wandernde Temperaturabweichung als eine Antwort des Systems auf ein definiertes Eingangssignal gemessen werden. Überdies kann die lokale Bluttemperatur über den Temperatursensor 14 kontinuierlich gemessen werden. Als Messort dient bevorzugt eine periphere Arterie 15, wie z. B. (wie dargestellt) die A. femoralis, A. radialis oder A. axillaris. Der arterielle Katheter 13 ist außerdem mit einer Druckmesslinie mit einen per se aus dem Stand der Technik bekannten Drucksensor 16 versehen, der den lokalen Druck in der peripheren Arterie 15 in Abhängigkeit von der Zeit misst.

Die Sensoren der Drucklinien 3,16 und die Temperatursensoren 4,15 sind mit einem Computersystem 17 verbunden, um die Eingangssignale, die entsprechenden Systemantworten sowie die Druck- und Temperatursignale in die Speichereinheit des Computersystems 17 zu überführen, wodurch diese gemessenen Daten damit zur weiteren Verarbeitung zur Verfügung stehen. Das Computersystem 17 verfügt über ein entsprechendes ausführbares Programm, welches die aufgezeichneten Datenwerte einlesen und aus den Datenwerten eine Vielzahl hämodynamischer Parameter bestimmen kann. Die erfindungsgemäßen Berechnungsmittel sind somit mittels geeigneter Programmroutinen implementiert. So wird insbesondere die vom arteriellen Temperatursensor 14 gemessene Temperatur als Thermodilutionskurve aufgezeichnet, welche der Systemantwort auf die Bolusinjektion durch den Injektionskanal 11 entspricht. Aus der Thermodilutionskurve 18 kann das Computersystem 17 über bekannte Algorithmen, wie z. B. die Steward-Hamilton Gleichung, das thermodilutionsbasierte Herzzeitvolumen (CO_{TD}) berechnen. Aus der Thermodilutionsmessung können ferner Parameter wie das globale enddiastolische Volumen (GEDV), das intrathorakale Blutvolumen (ITBV) und das extravaskuläre Thermovolumen (ETV) auf an sich bekannte Weise durch das Computersystem 17 bestimmt werden.

Aus der über den Sensor der arteriellen Drucklinie 16 aufgezeichneten arteriellen Druckkurve können mittels bekannter Algorithmen das Pulskontur-Herzzeitvolumen (PCCO), sowie Schlagvolumen (SV) und Schlagvolumen Variation (SW) bestimmt werden. Mittels des durch eine Thermodilutionsmessung bestimmten Referenz-CO_{TD} kann das PCCO kalibriert werden.

Das Computersystem 17 ist ferner über einen Steuerkanal 19 mit einer medizinischen Dosiervorrichtung im Injektionskanal 20 verbunden. Über die medizinische Dosiervorrichtung kann zur Thermodilutionsmessung eine automatische Bolusinjektion vorgenommen werden, alternativ kann auch ein Injektionskanal zur manuellen Bolusinjektion vorgesehen sein. Im Rahmen einer automatischen Kalibrierung wird vom Computersystem 17 über den Steuerkanal 19 ein Signal an die medizinische Dosiervorrichtung gegeben, woraufhin zur (Re-)Kalibrierung ein Flüssigkeitsbolus über den Zentralvenenkatheter 1 injiziert wird. Eingelesene Datenwerte und/oder berechnete Parameter, sowie Signale sind über das in das Computersystem 17 integrierte Display 18 vom Benutzer visuell erfassbar.

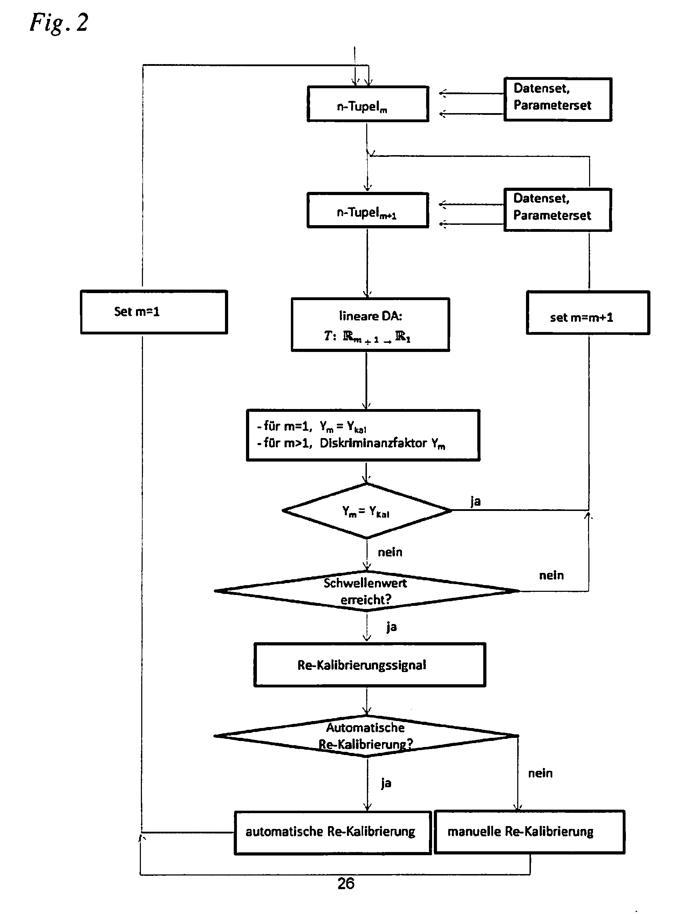

Figur 2 zeigt ein Flussdiagramm zur erfindungsgemäßen Einleitung einer Rekalibrierung in allgemeiner Form. Die mittels der in das Computersystem 17 integrierten Einlesemittel eingelesenen Datenwerte und/oder die mittels der in das Computersystem 17 integrierten Berechnungsmittel berechneten Parameter werden durch die ebenfalls in das Computersystem 17 integrierten Auslösemittel an einem ersten Zeitpunkt m in einen ersten n-Tupel mit n Komponenten zusammengefasst und bildet den n-Tupelₘ. Ein zweiter n-Tupel wird zu einem späteren zweiten Zeitpunkt, z.B. nach 5 Minuten, mittels der von den Einlesemitteln eingelesenen Datenwerte und/oder der mittels der Berechnungsmittel berechneten Parameter bestimmt und bildet den n-Tupelₘ₊₁. Aus der durch die Auslösemittel im nächsten Schritt durchgeführten linearen Diskriminanzanalyse wird der Diskriminanzfaktor Yₘ bestimmt. Dabei entspricht der erste nach einer Kalibrierung aus n-Tupel₁ und n-Tupel₂ bestimmte Diskriminanzfaktor Yₘ dem Referenz-Diskriminanzfaktor Y_{Kal}. Aus dem zu einem weiteren, dritten Zeitpunkt wie zuvor bestimmte n-Tupelₘ₊₁₊₁ (d.h. n-Tupel₃) und dem n-Tupel₁ wird mittels einer linearen Diskriminanzanalyse durch die Auslösemittel im nächsten Schritt der Diskriminanzfaktor Yₘ bestimmt. Dieser wird nun in einem nächsten Schritt zu dem Referenz-Diskriminanzfaktor Y_{Kal} in Relation gesetzt, z.B. in Form eines einfachen Vergleichs. Sind beide Werte gleich, i.e. Yₘ = Y_{Kal} = "ja", wird zu einem weiteren Zeitpunkt (m=3+1) von den Auslösemitteln wieder ein n-Tupel (d.h. n-Tupel₄) bestimmt und eine weitere lineare Diskriminanzanalyse durchgeführt. Sind beide Werte nicht gleich, i.e. Yₘ= Y_{Kal} = "nein", wird die Abweichung Yₘ von Y_{Kal}, z.B. relativ in Prozent oder absolut als Differenz, bestimmt und in einem nächsten Schritt verglichen, ob ein vorgegebener, relativer oder absoluter, Schwellenwert erreicht ist. So führt eine Abweichung der zuletzt bestimmten Diskriminanzgröße Y₃ um z.B. 15% von Y_{Kal} zum Auslösen eines Kalibrierungssignals durch die Auslösemittel, denn der Schwellenwert von 15% Abweichung ist erreicht ("ja"). Bei einer kleineren Abweichung der zuletzt bestimmten Diskriminanzgröße Y₃ um z.B. nur 1 % von Y_{Kal} wird hingegen kein Kalibrierungssignal durch die Auslösemittel ausgelöst ("nein"), sondern stattdessen ein weiterer n-Tupel bestimmt. Falls durch die Auslösemittel ein Kalibrierungssignal ausgelöst wurde, welches über das Display 18 z.B. als visuelle Anzeige "Calibrate!" vom Benutzer erfassbar ist, wird in einem nächsten Schritt bestimmt, ob eine automatische Rekalibrierung erfolgen soll. Eine automatische Rekalibrierung kann vom Benutzer vor Beginn der hämodynamischen Überwachung eingestellt werden, alternativ kann aber auch während einer laufenden hämodynamischen Überwachung von manueller auf automatische Rekalibrierung umgestellt werden. Nach einer Rekalibrierung werden die Zählerwerte (m) der n-Tupel wieder in den Ausgangszustand (m=1) zurückgesetzt. Alle zwischen zwei Kalibrierungen durch das Auslösesystem bestimmten n-Tupel und Diskriminanzgrößen werden durch die Speichereinheit des Computersystems 17 gespeichert.

Die Option einer automatischen Rekalibrierung entfällt, wenn anders als in der in Fig. 1 dargestellten Anordnung keine vom Computersystem 17 ansteuerbaren Mittel zur Bolusinjektion vorgesehen sind. Bei einer derartigen Ausführung der Erfindung muss der Benutzer die Kalibrierung manuell einleiten, wenn die Auslösung eines Kalibriersignals angezeigt wird.

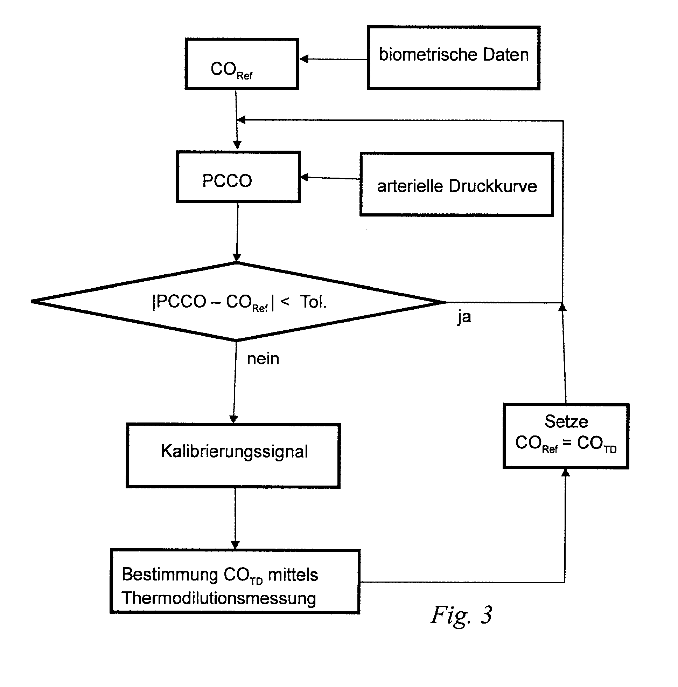

Figur 3 zeigt das Flussdiagramm der erfindungsgemäßen Einleitung einer Rekalibrierung am Beispiel eines 1-Tupels mit dem Pulskontur-Herzzeitvolumen (PCCO) als konstituierender Komponente. Zunächst erfolgt über die Eingabemittel, z.B. ein als Touchscreen ausgebildetes Display 18 das Eingeben spezifischer Informationen über den Patienten, wobei die spezifischen Informationen kategorisierende (z.B. Geschlecht und Alter) und biometrische Informationen (z.B. Körpergröße und -gewicht) enthalten und zur Erstellung des nominellen Herzzeitvolumens als Referenz-Herzzeitvolumens CO_{Ref} dienen. Alternativ kann auch ein Referenz-Herzzeitvolumen mittels Thermodilutionsmessung bestimmt werden.

Aus der arteriellen Druckkurve wird das Pulskontur-Herzzeitvolumen PCCO bestimmt. Solange dieses vom Referenz-Herzzeitvolumen um weniger als einen vorgegebenen Schwellenwert (Toleranzwert "Tol."), z.B. 10% oder 15% des Referenz-Herzzeitvolumens, abweicht, erfolgt die weitere Paramaterbestimmung ohne neuerliche Kalibrierung. Übersteigt die Abweichung |PCCO-CO_{Ref}| dagegen den Schwellenwert, wird ein Kalibrierungssignal ausgelöst.

Es kann auch vorteilhaft sein, unterschiedliche Schwellenwerte für eine Abweichung nach oben bzw. eine Abweichung nach unten festzulegen, d.h. das Kriterium für die Auslösung eines Kalibrierungssignals kann vorzeichenabhängig sein, d.h. dass eine Kalibrierung so lange unterbleibt wie gilt Tol1 < PCCO-CO_{Ref}| < Tol2. Gemäß einer lediglich als Zahlenbeispiel gewählten Ausführung kann ein Kalibrierungssignal ausgelöst werden, wenn die Differenz PCCO-CO_{Ref} den Bereich

- 0.1 CO_{Ref} < PCCO-CO_{Ref} < 0,15 CO_{Ref}

verlässt.

Die Auslösung eines Kalibrierungssignals kann dann angezeigt werden, um zu einer manuellen Kalibrierung aufzurufen, oder bei entsprechendem apparativer Ausstattung, wie in Fig. 1 dargestellt, eine automatische Kalibrierung auslösen. Das in der Kalibriermessung ermittelte Herzzeitvolumen CO_{TD} wird dann als neues Referenz-Herzzeitvolumen gesetzt.

Grundsätzlich kann die Bestimmung der Abweichung |PCCO-CO_{Ref}| dagegen "beat-to-beat" für jede PCCO-Messung erfolgen, es sind jedoch auch größere zeitliche Abstände der Bestimmung möglich. Soll vermieden werden, dass einzelne "Ausreißer", d.h. (isoliert) sowohl vom vorausgehenden als auch vom nachfolgenden Parameterwert stark (in dieselbe Richtung) abweichend bestimmte Parameterwerte eine Neukalibrierung auslösen, kann ein abgewandeltes Kalibrierkriterium verwendet werden. Als (ggf. auch zusätzliche) notwendige Bedingung für die Auslösung des Kalibriersignals kann z.B. festgesetzt werden, dass die Abweichung |PCCO-CO_{Ref}| bei mehreren, z.B. drei oder fünf, aufeinanderfolgend bestimmten Parameterwerten den Toleranzwert überschreiten muss, oder dass die Abweichung |PCCO - CO_{Ref}| bei einem Mindestanteil der zuletzt innerhalb einer vorgegebenen Zeitspanne ermittelten PCCO-Werte den Toleranzwert überschreiten muss (z.B. | PCCO - CO_{Ref} | > Tol. für mindestens 50% der innerhalb der letzten Minute ermittelten Werte). Oder aber es wird anstelle der Abweichung |PCCO-CO_{Ref}| die Abweichung des arithmetischen Mittels der letzten i Parameterwerte für das Kalibrierkriterium herangezogen so dass ein Kalibriersignal ausgelöst wird, wenn gilt

| [ (PCCOₘ+ PCCOₘ₋₁+ ... + PCCOₘ₋ᵢ) / i ] - CO_{Ref} | > Tol.

anstelle des Kalibrierkriteriums

| PCCO - CO_{Ref} | > Tol.

Eine entsprechende Filterung von Ausreißern" durch (ggf. auch gewichtete) Mittelwertbildung oder das Erfordernis eines Mindestanteils an Toleranzwertüberschreitungen innerhalb eines vorgegebenen Zeitintervalls kann vorteilhaft auch bei einer auf dem Vergleich von n-Tupeln mit n > 1 beruhenden Analyse sein.

Die Gefahr der Auslösung einer Kalibrierung aufgrund isoliert abweichender Parameterwerte kann auch durch Vorsehen eines zeitlichen Minimalabstandes zwischen zwei aufeinanderfolgenden Kalibrierungssignalauslösungen vermindert werden. Ein zeitlicher Minimalabstand zwischen zwei aufeinanderfolgenden Auslösungen des Kalibriersignals kann auch aus anderen Gründen vorteilhaft zu implementieren sein, beispielsweise um eine erhöhte Beanspruchung des Patienten und/oder des medizinischen Personals durch allzu häufige Anwendung eines invasiven Messverfahrens zu vermeiden.

Entsprechend kann der in Fig. 3 skiziierte Ablauf zu einem zweistufgen Entscheidungsablauf erweitert werden, wobei der Abfrage, ob | PCCO - CO_{Ref} | > Tol. oder | [ (PCCOₘ+ PCCOₘ₋₁+ ... + PCCOₘ₋ᵢ) / i ] - CO_{Ref} | > Tol. noch die Abfrage nachgeschaltet ist, ob der seit der letzten Kalibrierung vergangene Zeitraum eine vorgegeben Zeitspanne von z.B. 20 Minuten überschritten hat, und nur bei Überschreitung der Zeitspanne das Kalibrierungssignal ausgelöst wird.

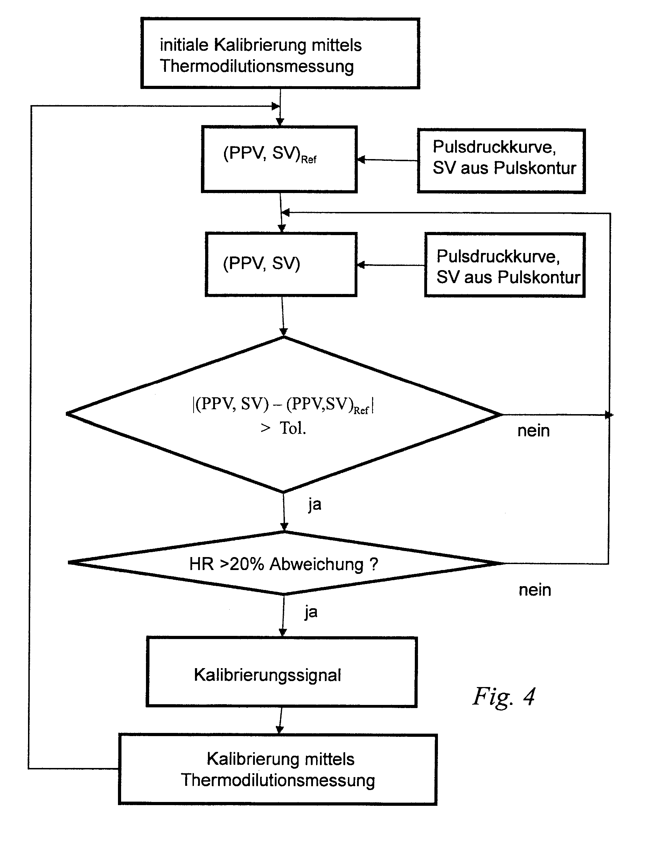

Die Figur 4 zeigt das Flussdiagramm der erfindungsgemäßen Einleitung einer Rekalibrierung am Beispiel eines 2-Tupels mit der Pulsdruckvariation (PPV) und dem Schlagvolumen (SV) als konstituierenden Komponenten. Die Auslösemittel bestimmen zu einem ersten Zeitpunkt mittels bekannter Algorithmen Schlagvolumen SV und Pulsdruckvariation PPV sowie ggf. den oder die weiteren interessierenden hämodynamischen Parameter. Der so gewonnene Referenztupel (PPV, SV)_{Ref} wird dann jeweils durch die Auslösemittel zum augenblicklich bestimmten (PPV, SV) in Relation gesetzt, z.B. durch Differenzenbildung und Vergleich mit einem Schwellenwert (Toleranzwert "Tol."). Ist die auch als Abstand im zweidimensionalen Vektorraum interpretierbare Abweichung | (PPV, SV) - (PPV, SV)_{Ref} | kleiner als der vorgegebene Schwellenwert, erfolgt die nächste Parameterbestimmung. Ist sie größer, so wird durch die Auslösemittel ein weiteres Kriterium abgefragt, z.B. die Abweichung der Herzfrequenz. Wenn diese Abweichung über einem bestimmten Wert liegt, z.B. > 20% von einem z.B. unmittelbar nach einer Kalibrierung bestimmten Wert, wird von den Auslösemitteln das Kalibrierungssignal ausgelöst, worauf sich die akkustische und/oder visuelle Anzeige des Kalibriersignals und/oder eine automatische Kalibrierung mittels Thermodilution anschließt. Der unmittelbar auf die Kalibrierung zu bestimmte Tupel (PPV; SV) dient nun wiederum als Referenztupel für die nachfolgenden Bestimmungen.

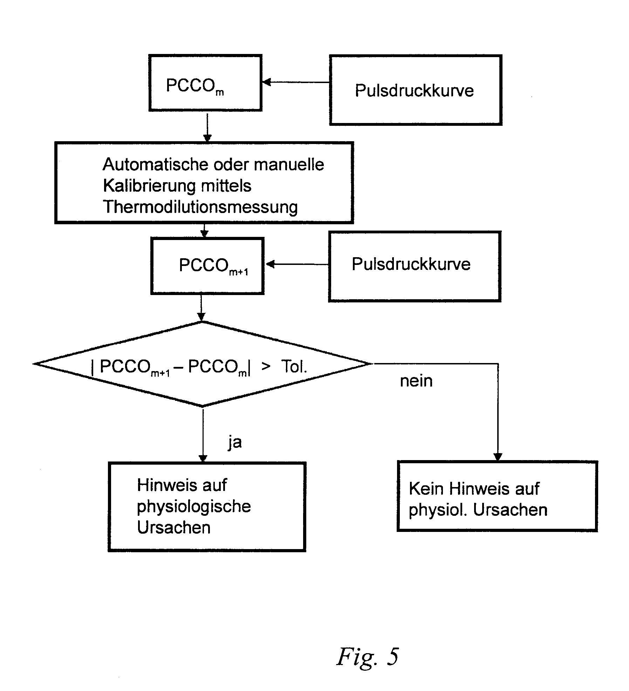

Figur 5 zeigt ein Flussdiagramm für die Bewertung des Kalibrierergebnisses gemäß einer bevorzugten Ausführungsform der Erfindung. Bei der Bewertung werden ein oder mehrere unmittelbar vor und unmittelbar nach einer manuellen oder automatischen Kalibrierung bestimmte(n) Werte eines oder mehrerer hämodynamischer Parameter zueinander in Relation gesetzt. Im Beispiel dargestellt ist ein einfacher Vergleich des vor und nach einer Kalibrierung erhobenen PCCO. Weichen die beiden Werte PCCOₘ₊₁- PCCOₘ um weniger als einen vorgegebenen Toleranzwert "Tol." voneinander ab, so wird dies dem Benutzer auf dem Display 18 angezeigt. Der Benutzer erkennt so, dass die Parameteränderung im Intervall zwischen den beiden letzten Kalibrierungen offenbar nicht auf Artefakte des verwendeten Algorithmus, eine Nulliniendrift oder dgl. zurückgeht, sondern physiologische Ursachen haben muss.

## Patentansprüche

1. Vorrichtung zur hämodynamischen Überwachung, wobei die Vorrichtung folgendes aufweist:
Einlesemittel zum wiederholten Einlesen von Daten, die mindestens eine physische Größe darstellen,
Berechnungsmittel zum Berechnen mindestens eines Parameters aus den eingelesenen Daten, und
Kalibriermittel zum Kalibrieren der Vorrichtung,
**dadurch gekennzeichnet, dass** die Vorrichtung Auslösemittel zum Auslösen eines Kalibrierungssignals in Abhängigkeit der zeitlichen Änderung der eingelesenen Daten und/oder mindestens eines der Parameter aufweist.

2. Vorrichtung gemäß Anspruch 1, wobei die Auslösemittel dazu ausgebildet sind, mittels einer uni- oder multivarianten Analyse eine Relation zu bilden zwischen mindestens einem zu einem ersten Zeitpunkt bestimmten n-Tupel mit n Komponenten, und mindestens einem zu mindestens einem entsprechenden späteren Zeitpunkt bestimmten n-Tupel mit n Komponenten, wobei n eine natürliche Zahl größer oder gleich 1 ist, und die Komponenten mindestens einen der Parameter und/oder einen Wert der eingelesenen Daten umfassen.

3. Vorrichtung gemäß einem der vorherigen Ansprüche, wobei die Auslösemittel dazu ausgebildet sind, mindestens eine mathematische Klassifizierungmethode auszuführen.

4. Vorrichtung gemäß Anspruch 3, wobei die Auslösemittel dazu ausgebildet sind, eine lineare Diskriminanzanalyse durchzuführen.

5. Vorrichtung gemäß einem der Ansprüche 2-4, wobei die n Komponenten mindestens einen Parameter umfassen ausgewählt aus der Gruppe Herzfrequenz (HR), Pulsdauer (T), Schlagvolumen (SV), mittlerer arterieller Druck (MAP), Pulskontur-Herzzeitvolumen (PCCO), Thermodilutions-Herzzeitvolumen (CO_{TD}), Schlagvolumenvariation (SW), systemischer Gefäßwiderstand (SVR), Pulsdruckvariation (PPV), Volumenreagibilitätsindex (FRI), ventrikuläre Kontraktilität (z. B. dPmx), atriale Drücke und/oder ventrikuläre Drücke (z. B. RAP, RVEDP, LVEDP), EKG-Abschnittsdaten, EKG-Intervalle, Kenndaten der die Hämodynamik beeinflussende Medikation und die Hämodynamik beeinflussende Atmungsparamater.

6. Vorrichtung gemäß einem der Ansprüche 2-5, wobei die n Komponenten mindestens einen Wert eingelesener Daten umfassen ausgewählt aus der Gruppe arterieller Druck (P), zentralvenöser Druck (CVP), Bluttemperatur (Tb), periphere Sauerstoffsättigung (Sp02), zentralvenöse Sauerstoffsättigung (Scv02).

7. Vorrichtung gemäß einem der vorangehenden Ansprüche, wobei die Auslösemittel zum Berücksichtigen innerhalb eines vorgegebenen Intervalls aufeinanderfolgender gegenläufiger zeitlichen Änderungen der eingelesenen Daten und/oder mindestens eines der Parameter ausgebildet sind.

8. Vorrichtung gemäß einem der vorangehenden Ansprüche, welche Mittel zum visuellen und/oder akustischen Anzeigen der Auslösung des Kalibrierungssignals aufweist.

9. Vorrichtung gemäß einem der vorangehenden Ansprüche, wobei das Kalibrierungssignal an die Kalibriermittel zur Einleitung einer automatischen Kalibrierung oder Rekalibrierung übertragen wird.

10. Vorrichtung gemäß einem der vorangehenden Ansprüche, wobei die Kalibrierung eine Thermodilutionsmessung umfasst.

11. Vorrichtung gemäß einem der vorangehenden Ansprüche, wobei die Auslösemittel zur zwei- oder mehrstufigen Prüfung der Abhängigkeit der zeitlichen Änderung der eingelesenen Daten und/oder mindestens eines der Parameter ausgelegt sind,
und die zwei- oder mehrstufige Prüfung die hierarchische Überprüfung mindestens zweier das Auslösen des Kalibrierungssignals bedingender, von der zeitlichen Änderung der eingelesenen Daten und/oder mindestens eines der Parameter abhängender Auslösekriterien umfasst.

12. Vorrichtung gemäß einem der vorangehenden Ansprüche, wobei die Vorrichtung ferner Bewertungsmittel aufweist zum Bewerten einer arithmetischen Relation zwischen mindestens einem unmittelbar vor einer Kalibrierung und mindestens einem unmittelbar nach dieser Kalibrierung ermittelten Werts der eingelesenen Daten und/oder zwischen mindestens einem der unmittelbar vor einer Kalibrierung und unmittelbar nach dieser Kalibrierung bestimmten Parameters.

13. Vorrichtung gemäß einem der vorangehenden Ansprüche, wobei die Vorrichtung ferner Eingabemittel zum Eingeben biometrischer und/oder kategorisierender Informationen über den Patienten umfasst.

14. Vorrichtung gemäß Anspruch 13, welche Anpassmittel zum Anpassen eines das Auslösen des Kalibrierungssignals in Abhängigkeit der zeitlichen Änderung der eingelesenen Daten und/oder mindestens eines der Parameter bedingenden Kriteriums in Abhängigkeit eingegebener biometrischer und/oder kategorisierender Informationen über den Patienten aufweisen.

15. Verfahren zur Kalibrierung einer Vorrichtung zur hämodynamischen Überwachung eines Patienten, aufweisend
(a) Einlesen von Daten, die mindestens eine physische Größe darstellen
(b) Berechnen mindestens eines Parameters aus den eingelesenen Daten,
(c) Auslösen eines Kalibrierungssignals in Abhängigkeit der zeitlichen Änderung der eingelesenen Daten und/oder des mindestens einen Parameters
(d) Kalibrieren der Vorrichtung in Abhängigkeit des Kalibrierungssignals,
